# DEMANDE DE BREVET EUROPEEN

(11) **EP 1 133 972 A1**
(43) Date de publication de la demande: **19.09.2001**
(21) Numéro de dépôt: 01400676.1
(22) Date de dépôt: 14.03.2001
(51) Int. Cl.: A61K 6/00, A61K 6/097, A61K 6/08

(54) **Prothèse dentaire à surface hydrophile et procédé d'obtention**

(30) Priorité: 15.03.2000 FR 0003329
(71) Demandeur: Association pour les transferts de technologie Du Mans, 72000 Le Mans (FR); Bellessort, Stéphane, 7200 Le Mans (FR)
(72) Inventeur: Bellessort, Stéphane, 72000 Le Mans (FR); Legeay, Gilbert, 72650 Saint Saturnin (FR)
(74) Mandataire: Michelet, Alain

(57) **Abrégé**

La présente invention est donc relative à une prothèse dentaire comprenant un premier matériau polymère de base contenant des atomes d'hydrogène, à la surface duquel les atomes d'hydrogène sont remplacés partiellement par des groupements - OH, - COOH, - C=O, - OOH, - NH2,-C=NH et/ou - CONH2 par un traitement par plasma, caractérisée en ce que ladite surface du premier matériau polymère est recouverte au moins sur certaines zones d'une couche d'un second matériau polymère à caractère hydrophile, lié de manière non covalente au premier matériau polymère.

## Description

La présente invention est relative à des prothèses dentaires améliorées ainsi qu'à un procédé d'obtention de telles prothèses.

La France compte 10.000.000 de porteurs de prothèses dentaires adjointes et 4.500.000 de porteurs de prothèses amovibles complètes. En extrapolant à la Communauté Européenne, le nombre de porteurs de prothèses adjointes se situe vraisemblablement aux alentours de 60.000.000 d'individus, dont 25.000 000 de porteurs de prothèses amovibles complètes.

Les prothèses dentaires en résine, en particulier en poly(méth)acrylate ont une énergie de surface intermédiaire comprise entre 35 et 45 mJ par m². Du fait de cette énergie et de la porosité de ce matériau, ces prothèses présentent deux inconvénients majeurs :
- une adhésion importante des protéines de l'alimentation qui provoque : candidoses, caries, et stomatites prothétiques, et
- une rétention insuffisante des appareils complets et principalement des appareils complets inférieurs.

Divers moyens ont été employés pour améliorer la rétention de ces prothèses. On a par exemple cherché à améliorer les prothèses par polymérisation de fibres polymères hydrophiles telles que le (meth)acrylkoxy groupé, comme décrit dans la demande de brevet européen N° EP 0 401.385. On a aussi cherché à recouvrir le poly(méth)acrylate par du dioxyde de silicium. Néanmoins, un tel procédé s'est révélé onéreux et les prothèses en résultant étaient instables.

Les intrados prothétiques des prothèses dentaires complètes ont été aussi soumis à un sablage au quartz pendant 30 à 60 secondes, ou encore à un sablage à l'aide d'une poudre d'alumine d'un diamètre moyen de 50 µm.

Cette technique s'est révélée elle aussi inadaptée, du fait de l'augmentation considérable de la colonisation bactérienne.

Des adhésifs dentaires sont aussi utilisés afin d'assurer le maintien en bouche des prothèses dentaires. Néanmoins, ces adhésifs nécessitent d'être utilisés une ou plusieurs fois par jour. De plus, ils représentent un coût important et sont mal considérés par les praticiens.

Selon le brevet français N°96.06680 publié sous le N°FR 2.749.170, un accroissement de l'énergie de surface des prothèses et une réduction concomitante de la plaque dentaire sont obtenus par traitement du matériau polymère de base de ces prothèses par un plasma d'argon, d'azote, de gaz carbonique ou d'oxygène. Les prothèses ainsi traitées présentent par rapport au polymère de méthacrylate de méthyle des angles de contact de l'eau fortement réduits après traitement de ce polymère par plasma.

En outre, est également observé un développement limité de la plaque dentaire au niveau de l'intrados prothétique .

Il s'est avéré que les caractéristiques d'énergie de surface des prothèses conformes à l'enseignement du brevet français précité pouvaient encore être améliorées. En outre, les propriétés d'adhésion de l'intrados prothétique de telles prothèses avaient tendance à diminuer au cours du temps par usure mécanique, à la suite des entretiens quotidiens de la prothèse par brossage.

Le demandeur s'est donc attaché à mettre au point des prothèses ayant une énergie de surface accrue par rapport aux prothèses décrites dans l'état de la technique, et dont l'énergie de surface reste sensiblement constante durant une grande partie, sinon la totalité, du temps d'utilisation de ces prothèses.

II a été montré, selon l'invention, que le revêtement par une couche de polymère hydrophile du polymère de base constitutif d'une prothèse, préalablement traité par plasma, permettait de résoudre ces problèmes.

La présente invention est donc relative à une prothèse dentaire comprenant un premier matériau polymère de base contenant des atomes d'hydrogène, à la surface duquel les atomes d'hydrogène sont remplacés partiellement par des groupements - OH, - COOH, - C=O, - OOH, - NH2, - C=NH et/ou - CONH2 par un traitement par plasma, caractérisée en ce que ladite surface du premier matériau polymère est recouverte au moins sur certaines zones d'une couche d'un second matériau polymère à caractère hydrophile, lié de manière non covalente au premier matériau polymère.

De manière préférée, le traitement préalable du premier matériau polymère constitutif de la prothèse par un plasma d'oxygène, d'argon, d'azote ou de gaz carbonique, est réalisé conformément à l'enseignement du brevet français FR 2.749.170. Le traitement par plasma permet l'obtention d'un matériau polymère de base constitutif de la prothèse, présentant une teneur en oxygène, sur une épaisseur de 10 à 30 nm à la surface dudit matériau, augmentée de 1 à 15% et encore plus préférentiellement de 1 à 10% par rapport à un polymère non traité préalablement par plasma. En créant des sites oxydés, le traitement de surface par plasma augmente sensiblement l'énergie de surface du premier matériau polymère permettant le dépôt du second matériau polymère à caractère hydrophile. En outre, l'augmentation de la teneur en oxygène de la surface du premier matériau polymère permet la création de liaisons de type hydrogène entre ce premier matériau polymère et la couche du second matériau polymère à caractère hydrophile. Ainsi, l'adhésion entre le premier matériau polymère à la couche du second matériau polymère est maximale sans nécessiter de greffage par liaison covalente des polymères entre eux.

Les essais réalisés par le demandeur ont montré qu'aucun décollement de la couche du second matériau polymère n'est observé, même après plusieurs dizaines de lavages prolongés suivis de séchages.

Par polymère hydrophile au sens de l'invention, on entend un polymère capable d'être dissout par une solution aqueuse comprenant au minimum 80% en poids d'eau.

De manière tout à fait préférée, le second matériau polymère à caractère hydrophile est au moins partiellement soluble dans l'eau ou dans un solvant totalement miscible à l'eau.

De préférence, ce second matériau polymère à caractère hydrophile se gonfle et devient déformable au contact de l'eau.

Le second matériau polymère est préférentiellement choisi parmi les celluloses et leurs dérivés, les polyacrylamides et leurs copolymères, la polyvinylpyrrolidone ( PVP ) et ses copolymères, les copolymères de l'acétate de vinyle et de l'alcool vinylique, les polyéthylèneglycols, les propylèneglycols, les poly(méth)acrylates hydrophiles, les polyosides et les chitosans.

Le second matériau polymère peut aussi être un mélange de plusieurs des polymères ci-dessus, en général un mélange de deux ou de trois des polymères ci-dessus.

La couche du second matériau polymère a une épaisseur comprise entre environ 2 et environ 500 µm, préférentiellement entre environ 5 et environ 100 µm, et de manière tout à fait préférée entre environ 5 et environ 50 µm.

En général, la couche du second matériau polymère recouvre au moins une partie, de préférence la totalité de la surface de l'intrados prothétique.

Dans un premier mode de réalisation préféré, le second matériau polymère recouvre exclusivement l'intrados prothétique de ladite prothèse.

Dans un second mode de réalisation préféré, le second matériau polymère recoure à la fois l'intrados et l'extrados prothétique.

La couche du second matériau polymère telle que définie ci-dessus, lorsqu'elle est au contact de la gencive, est fortement gonflée. Cette couche visqueuse, malléable et déformable permet le maintien de la prothèse sur le palais ou la gencive avec une tenue comparable à celui observé avec un adhésif classique utilisé en dentisterie.

L'adhésion particulièrement grande des prothèses dentaires selon l'invention sur les tissus de la gencive ou du palais résulte d'une combinaison des propriétés physico-chimiques hydrophiles de la couche du second matériau polymère à forte énergie de surface et des propriétés purement mécaniques dues au caractère malléable et déformable de la couche du second matériau polymère permettant d'épouser étroitement les irrégularités des tissus de la gencive ou du palais.

Pour obtenir une telle combinaison d'effets physico-chimiques et mécaniques, il est nécessaire que la couche du second matériau polymère ait une épaisseur minimale d'au moins 2 µm, et de préférence 5µm, ou davantage, notamment 10 µm. La limite supérieure de 500 µm de l'épaisseur de la couche du second matériau polymère ne saurait être dépassée sans entraîner simultanément un inconfort significatif pour le patient, dû à une pression trop importante de la prothèse sur la surface d'appui et des risques concomitants d'irritation, voire de blessure, des tissus gingivaux.

En outre, le demandeur a observé que l'utilisation des prothèses dentaires selon l'invention réduisait considérablement les risques d'infections bactériennes du fait d'une moindre colonisation du matériau par les bactéries associée à un contact très étroit entre les tissus gingivaux et la couche du second matériau polymère de la prothèse, entraînant une immobilisation quasi-totale, et sans frottement, de la prothèse en place; l'irritation des tissus, observée avec les prothèses de l'état de la technique, est ainsi réduite.

La prothèse dentaire selon l'invention est de préférence une prothèse définitive, neuve ou ancienne mais elle peut aussi être appliquée à des prothèses à pose immédiate.

Un avantage supplémentaire de la présence de la couche du second matériau polymère de la prothèse dentaire selon l'invention est une facilité d'entretien, l'usure mécanique provoquée par un entretien par brossage au moins une ou deux fois par jour étant compensée d'une part, par l'épaisseur minimale de la couche de matériau polymère hydrophile et, d'autre part, par sa capacité à se déformer, en conservant ainsi un contact étroit avec les tissus gingivaux quels que soient le degré d'usure et le temps d'utilisation de la prothèse.

Selon une autre caractéristique d'une prothèse dentaire selon l'invention, le remplacement partiel des atomes d'hydrogène à la surface du premier matériau polymère est localisé sur une épaisseur de 10 à 30 nm.

Selon encore une autre caractéristique, le traitement par plasma peut être réalisé indifféremment par mise sous vide dans une machine à plasma, par mise sous vide partielle avec un gaz tel que l'oxygène, l'argon, l'azote ou le gaz carbonique, par passage d'une décharge électrique, par exemple à la fréquence de 13,56 MHz pendant plusieurs minutes, provoquant un bombardement de la surface du premier matériau polymère par le gaz excité et donc une modification chimique en surface du polymère.

L'invention a également pour objet un procédé d'obtention d'une prothèse dentaire comprenant une étape de traitement en surface par plasma d'oxygène, d'argon, d'azote ou de gaz carbonique d'un premier matériau polymère, préalablement obtenu sous la forme appropriée à son utilisation, caractérisé en ce que la surface du premier matériau polymère traité est recouverte d'un second matériau polymère à caractère hydrophile, qui se lie de manière non covalente au premier matériau polymère.

De préférence, le traitement par un plasma d'argon, d'azote, de gaz carbonique ou d'oxygène est réalisé à une puissance d'émission comprise entre 1 et 10 watts et préférentiellement 2 et 5 watts par litre de capacité du réacteur.

Les traitements à l'argon ou à l'azote sont plus particulièrement adaptés, car ils évitent la formation de groupements à oxydation rapide à la surface des prothèses.

De préférence, le temps de traitement dans le réacteur est avantageusement compris entre 2 et 20 minutes, préférentiellement entre 5 et 15 minutes. La pression, quant à elle, se situe avantageusement entre 0,01 et 10 Torr et préférentiellement entre 0,1 et 1 Torr.

De manière avantageuse, le premier matériau polymère constituant la base de la prothèse est un polyméthacrylate d'éthyle, un polyméthacrylate de méthyle (PMMA), éventuellement réticulé, ou un mélange de ces deux polymères, ou encore d'autres dérivés acryliques. Il peut être aussi un polymère à base de polydiméthylsiloxane.

Il peut aussi être tout autre polymère ayant des qualités mécaniques et chimiques compatibles avec la fabrication de prothèses dentaires, et présentant en surface des atomes d'hydrogène susceptibles d'être substitués par d'autres atomes, groupements ou fonctions après traitement à l'aide de plasmas froids.

Les traitements par les plasmas froids sont effectués selon des méthodes connues de l'homme du métier. A cet effet, il pourra se référer au manuel général suivant : " Plasmas réactifs ; A. RICARD, 1995, Edition de la Société Française du Vide".

Selon un mode de réalisation préférentielle du procédé ci-dessus, le second matériau polymère est apporté à la surface du premier matériau sous la forme d'une solution ou suspension aqueuse dont la concentration en polymère est comprise environ entre 0,5% et 5%, de préférence entre environ 1% et environ 3% en poids total de la solution ou suspension aqueuse.

Selon un mode de réalisation particulier du procédé, la solution aqueuse du second matériau polymère contient, outre de l'eau, une faible proportion d'un solvant miscible dans l'eau tel que l'éthanol ou l'acétone, ce solvant étant soit distillé soit purifié, par exemple par passage sur des résines échangeuses d'ions, soit encore filtré sur membrane, par exemple un filtre de taille de pore de 0,22 µm de la marque Millipore.

Selon un autre aspect, la solution aqueuse contient soit un seul polymère à caractère hydrophile, soit un mélange de plusieurs des polymères hydrophiles partiellement ou totalement hydrosolubles tels que ceux définis dans la présente description.

La solution aqueuse contenant le polymère hydrophile ou le mélange de polymères hydrophiles peut également contenir un ou plusieurs additifs destinés à améliorer son étalement sur la surface du premier matériau polymère préalablement traité par plasma . De tels additifs peuvent être des tensio-actifs neutres ou à caractère acide ou basique. On préfère toutefois des additifs neutres tels que les polyéthylène et les polypropylène glycols, le polyoxyéthyle sorbitol stéarate (Tween®), un polyéther ou encore un polysiloxane-polyéther.Ces produits sont notamment commercialisés par la Société TEGO Chemie ou BYK Chemie.

Le cas échéant, la solution aqueuse du polymère hydrophile ou du mélange du polymères hydrophiles, contient également un ou plusieurs agents antiseptiques, antibactériens, ou conservateurs permettant d'éviter la prolifération bactérienne, soit dans la solution aqueuse de polymère hydrophile avant utilisation, soit au moment du dépôt, sur le support de prothèse.

La solution aqueuse de polymères hydrophiles peut également comprendre un agent promoteur d'adhérence afin d'améliorer la tenue de la prothèse sur le tissu buccal, tel qu'un organosilicié, un phosphate, un phosphoaluminate ou un zircoaluminate.

De préférence, la solution aqueuse de polymère hydrophile ou du mélange de polymères hydrophiles peut être stérilisée ou pasteurisée afin d'assurer une bonne conservation.

Enfin, on peut également ajouter à la solution aqueuse de polymère hydrophile un agent de durcissement capable d'améliorer la résistance de la couche du second matériau polymère à l'abrasion, tel que par exemple la silice.

La concentration en polymère(s) hydrophile(s) dans la solution aqueuse ci-dessus sera aisément adaptée par l'homme du métier en fonction du résultat désiré. Plus la concentration de la solution aqueuse en matériau polymère hydrophile est importante, plus l'épaisseur de la couche du second matériau polymère hydrophile appliquée à la surface traitée du premier matériau polymère est grande.

De préférence, le second matériau polymère hydrophile est apporté à la surface du premier matériau polymère par trempage ou encore par application au pinceau ou au pistolet.

Pour une application par trempage, la prothèse de base constituée du premier matériau polymère préalablement traité par plasma est trempée quelques secondes dans la solution aqueuse de polymère hydrophile puis est égouttée et enfin séchée à l'air ambiant ou encore en étuve ventilée pendant une durée de 10 à 40 minutes.

Une application à l'aide d'un pinçeau ou d'une brosse peut avoir l'intérêt de permettre une sélection des zones de dépôt du second matériau polymère hydrophile. Dans ce mode de réalisation, le séchage est réalisé comme décrit ci-dessus.

Lorsque le second matériau polymère hydrophile est appliqué à l'aide d'un pistolet, un film sec est obtenu plus rapidement qu'avec les autres techniques. Cependant, un séchage complémentaire tel que décrit ci-dessus est souhaitable.

Plusieurs applications successives de la solution aqueuse de polymère hydrophile peuvent être effectuées si l'on veut obtenir une grande épaisseur de revêtement.

Le procédé tel que défini ci-dessus peut être appliqué indifféremment à des prothèses dentaires amovibles ou mobiles, de préférence des prothèses définitives, que ces prothèses soient neuves ou encore qu'il s'agisse de prothèses ayant déjà été portées, auquel cas le procédé selon l'invention permet un rajeunissement de telles prothèses.

Les prothèses dentaires selon l'invention sont conservées, transportées et manipulées sans précautions particulières. Elles peuvent subir un entretien par brossage régulier avec des brosses souples et une pâte dentifrice classique sans subir de détérioration significative.

La présente invention est en outre illustrée, sans pour autant être limitée, par la figure et les exemples suivants :
La figure unique illustre des clichés photographiques d'une goutte sessile sur respectivement:

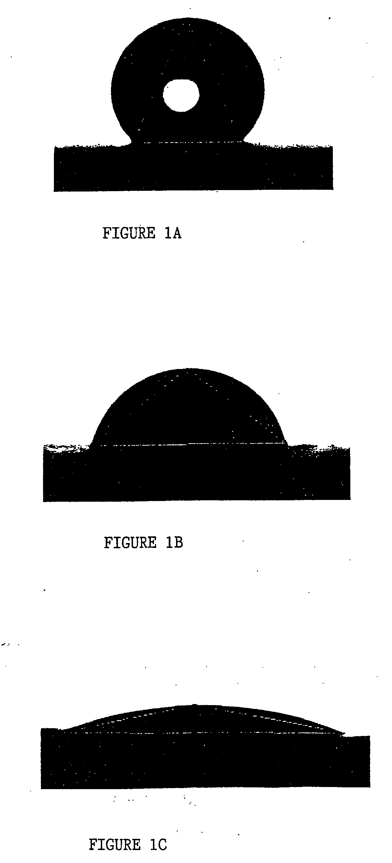

Figure 1A: la surface hydrophobe d'un matériau support de prothèse en polyméthacrylate de méthyle (PMMA) traité par plasma d'argon fluoré (Angle de contact de 131°).
Figure 1B: la surface d'un matériau support de prothèse en PMMA non traité (Angle de contact de 76°).
Figure 1C: surface d'un matériau support de prothèse traitée par plasma d'Argon après dépôt d'une couche de polyvinyl pyrrolidone (Angle de contact de 22°).

### Exemples

### Matériel et méthodes

### 1. Mesure du mouillage

L'indice de mouillage est donné par la valeur de l'angle (theta) pris au point de raccordement d'une goutte de liquide avec la surface sur laquelle il est déposé. Le chiffre correspond à cet angle déterminé d'un côté par la droite correspondant à la surface de l'objet, et de l'autre côté, par la tangente à la goutte au point de raccordement goutte/surface du support. Cette mesure est illustrée sur le dessin annexé.

Pour une surface hydrophile : la goutte est plate : l'angle est faible ; (figure 1C);

Pour une surface hydrophobe : la goutte ressemble à une bille : l'angle est élevé. (Figures 1A et 1 B).

### Origine de l'eau

L'eau utilisée pour les mesures est :
- soit déminéralisée par des résines échangeuses d'ions ;
- soit distillée fraîchement ;
- soit d'origine commerciale : préparation injectable ( ppi ), eau pure.

Cette eau doit être conservée dans des récipients fermés en quartz et à l'abri de la lumière et de la chaleur.

### Dépôt de la goutte

Le volume de la goutte est de quelques microlitres. Dans ces conditions, le poids de la goutte est faible, de sorte que la goutte ne se déforme pas de façon importante sous l'effet de la gravité. Cette goutte peut être produite soit avec une micropipette (type Pasteur), soit avec une microseringue.

### Appareil utilisé

La taille de la goutte ne permet pas de mesurer directement l'angle de contact. Il est nécessaire d'utiliser un appareil optique. Sont proposés :
- un appareil photographique avec objectif macro ;
- un système de projection sur un écran disposant d'un système de graduation des angles ;
- un goniomètre à lunette grossissante : on trouve sur le marché des équipements tels que ceux commercialisés par la société Ramé-Hart aux Etats-Unis, par la société Kruss en Allemagne ou par la société Kyowa au Japon ;
- un goniomètre avec caméra et informatique commercialisé par la société Kruss ou par la société GBX Instruments en France (Digidrop).

Il est souhaitable que l'équipement soit localisé dans un local à une température constante, proche de 20°C.

### Les mesures présentées dans les exemples ci-après sont faites avec l'équipement Digidrop

La goutte est formée à l'extrémité de la seringue (ou de la micropipette), puis le support est rapproché lentement de la goutte (goutte sessile). L'image de la goutte et de la surface apparaît immédiatement sur l'écran : le travail de mesure de l'angle se fait sur cette image.

La mesure est faite soit automatiquement, soit de façon manuelle en pointant avec la souris de l'ordinateur les deux points de raccordement goutte/surface ainsi que le sommet de la goutte: l'ordinateur calcule automatiquement la valeur de l'angle de contact. Il est préférable de réaliser la mesure de façon manuelle, des problèmes de reflets viennent en effet parfois perturber complètement la lecture de l'image par la caméra lorsque celle-ci travaille en mode automatique.

La mesure est effectuée environ 5 secondes après le dépôt de la goutte. En effet, dans le cas de certaines surfaces hydrophiles, un étalement progressif au cours du temps peut être constaté. Par ailleurs, une évaporation de l'eau a lieu, ce qui n'est cependant pas perceptible dans les 5 secondes après le dépôt.

Pour chaque surface, un minimum de trois mesures est réalisé et la valeur moyenne est calculée ainsi que la valeur de l'écart type.

Les images et les valeurs des angles sont enregistrées par l'ordinateur.

### 2. Traitement du premier matériau polymère par plasma

Pour l'ensemble des exemples qui suivent, des prothèses en résine polyacrylique, obtenue à partir de plusieurs monomères acryliques, avec une charge minérale, et couramment commercialisées, ont été traitées dans une machine à plasma de 20 litres de capacité émettant une fréquence de 13,56 MHz, en présence d'argon.

La prothèse, avant traitement, est propre et sèche. Le cas échéant, un séchage est réalisé par un passage dans une étuve ventilée ou encore dans le courant d'air chaud d'un sèche-cheveux.

Le traitement par plasma est réalisé par exposition d'une prothèse complète pendant 10 minutes à une puissance de décharge de 50 watts.

### Exemple 1- Prothèses recouvertes de polyvinylpyrrolidone.

Une solution aqueuse à 1% de PVP (Kollidon 30 de BASF/Laserson, pureté pharmaceutique) est préparée.

Après traitement par plasma, la prothèse est trempée quelques secondes dans la solution aqueuse à 1% de PVP précédente, puis retirée, égouttée et séchée soit à l'étuve, soit au sèche-cheveux, soit par flux d'air éventuellement chauffé.

Des mesures de mouillage sont effectuées par la technique de la goutte d'eau et mesure de l'angle de contact.

Les résultats sont les suivants :
1a) prothèse non traitée : valeur d'angle : 80° à 95°
1b) prothèse traitée plasma : valeur d'angle : 44° à 51°
1c) prothèse selon l'invention (prothèse 1b avec dépôt PVP) : 16° à 18°

### Exemple 2- Prothèses recouvertes d'hydroxypropylméthylcellulose.

Une solution aqueuse d'hydroxypropylméthylcellulose (E4M de Dow Chemical) à 1% est préparée.

Après traitement par plasma, le trempage, l'égouttage, le séchage puis le mouillage sont réalisés. Les valeurs d'angle sont les suivantes :
2a) prothèse non traitée : 80° à 95
2b) prothèse traitée plasma : 38° à 45°
2c) prothèse selon l'invention (prothèse 2b avec dépôt E4M) : 60° à 78°

### Exemple 3 : Etude comparative des indices de mouillage de plusieurs prothèses vieillies artificiellement, après séchage à l'étuve.

Des prothèses en résine polyacrylique ont été traitées pendant 10 minutes dans une machine à plasma de 20 litres de capacité émettant une fréquence de 13,56 Mhz en présence d'argon, réglée sur la puissance 50 watts, comme décrit dans la section Matériel et Méthodes ci-dessus.

Après traitement par plasma, certaines prothèses ont été recouvertes d'une couche de polymère hydrophile, soit de PVP (solution à 1%) soit de HPMC (solution à 0,2%), selon les techniques décrites respectivement aux exemples 1 et 2 ci-dessus.

Ensuite, les différentes prothèses ont été laissées à sec ou ont subi une étape de trempage pendant 21 jours dans de l'eau distillée ou dans une solution saline (0,9% en poids de NaCI).

Après trempage, les prothèses sont séchées pendant 1 heure à 50°C et sont refroidies à température ambiante.

Les résultats des mesures de mouillage (Angle théta) sont représentés dans le tableau 1.

Pour les prothèses conservées à sec, les valeurs d'angle des prothèses traitées conformément à l'invention sont significativement inférieures à celles observées après simple traitement par plasma. En particulier, une valeur d'angle de 55,1 est obtenue pour la prothèse simplement traitée par plasma, alors qu'un traitement par le polymère hydrophile polyvinyl pyrrolidone (PVP) permet de réduire la valeur d'angle à 28,4.

Ces résultats montrent l'hydrophilicité accrue des prothèses traitées conformément à l'invention, par rapport aux prothèses connues antérieurement.

Pour les prothèses conservées dans de l'eau distillée ou dans la solution saline (0,9% en poids de NaCI), les valeurs d'angle de mouillage des prothèses conformes à l'invention (PVP et HPMC) sont du même ordre que celles observées avec les prothèses n'ayant subi que le traitement par plasma.

Ces résultats montrent que dans des conditions physiologiques, les prothèses conformes à l'invention ont une énergie de surface au moins du même ordre que les prothèses de l'état de la technique.

En outre, comme déjà détaillé dans la description, les prothèses selon l'invention, de par leur malléabilité, ont des propriétés mécaniques avantageuses, absentes dans les prothèses de l'état de la technique.

### EXEMPLE 4 - Etude comparative des indices de mouillage de plusieurs prothèses vieillies artificiellement après séchage à l'air libre.

Des prothèses ont été fabriquées comme décrit à l'exemple 3 ci-dessus.

Des essais ont été réalisés pour étudier la conservation des prothèses après un stockage pendant trois semaines à 20°C, soit à sec, soit dans de l'eau pure, soit en solution saline (0,9% en poids de NaCI), puis épongeage à l'aide de papier absorbant et séchage à l'air libre pendant quelques minutes.

Un tel séchage superficiel maintient un film d'eau dans les irrégularités de surface du matériau support de la prothèse. De ce fait, la reproductibilité des mesures d'angle est moins grande que lorsque le matériau support de prothèse est traité à l'étuve conformément à l'exemple 3.

Néanmoins, les résultats présentés permettent un suivi dans le temps des caractéristiques physico-chimiques des matériaux supports traités ou non traités.

Les résultats obtenus après trois semaines de vieillissement accéléré sont présentés ci-dessous:

### Prothèses non traitées.

Les valeurs d'angles sont respectivement de :
- 84° à sec
- 92° dans l'eau distillée
- 80° dans l'eau saline.

En moyenne les plaques ont un mouillage compris entre 80° et 92°.

### Prothèses traitées par plasma d'Argon

Les valeurs d'angles sont respectivement de :
- 55° à sec
- 70° dans l'eau distillée
- 52° dans l'eau saline.

En moyenne, les plaques ont un mouillage compris entre 52° et 70°.

### Prothèses traitées par plasma + PVP

Les valeurs d'angles sont respectivement de :
- 28° à sec
- 75° dans l'eau distillée
- 56° dans l'eau saline.

En moyenne, les plaques ont un mouillage compris entre 28° (à sec) et 75°.

### Prothèses traitées par plasma + HPMC

Les valeurs d'angles sont respectivement de :
- 45° à sec
- 76° dans l'eau distillée
- 59° dans l'eau saline.

En moyenne, les plaques ont un mouillage compris entre 45° et 76°.

En outre, les indices de mouillage (valeur d'angle théta) ont été suivi respectivement au temps 0, et après 7, 14 ou 21 jours de durée du traitement de vieillissement accéléré décrit ci-dessus.

Les résultats sont représentés dans le tableau 2.

En premier lieu, l'ensemble des résultats de vieillissement montre que l'hydrophilicité des prothèses revêtues d'une couche de polymère hydrophile selon l'invention ont des propriétés d'hydrophilicité au moins du même ordre que celles des prothèses de l'état de la technique.

Concernant les prothèses conservées à sec ou dans de l'eau distillée, il est observé avec les prothèses selon l'invention, une hydrophilicité significativement plus grande que celle obtenue avec les prothèses de l'état de la technique, y compris les prothèses ayant subi un traitement par plasma, et ceci quel que soit le polymère hydrophile employé.

En second lieu, les propriétés d'hydrophilicité des prothèses selon l'invention sont sensiblement constantes au cours du temps, alors qu'elles diminuent considérablement au cours du temps avec les prothèses de l'état de la technique. Ces propriétés supérieures des prothèses selon l'invention sont particulièrement observées après vieillissement à sec ou dans de l'eau distillée.

**TABLEAU 1**

| Référence des plaques | Traitement* | Conservation | Mouillage après séchage 1H |
|---|---|---|---|
| 21-1a | Témoin | A sec | 82,9 |
| 21-2a | Plasma | A sec | 55.1 |
| 21-3a | Plasma + PVP | A sec | 28.4 |
| 21-4a | Plasma+HPMC | A sec | 45.3 |
| | | | |
| 21-1n | Témoin | Eau distillée | 91.9 |
| 21-2b | Plasma | Eau distillée | 70.4 |
| 21-3b | Plasma + PVP | Eau distillée | 69.8 |
| 21-4b | Plasma+HPMC | Eau distillée | 76.6 |
| | | | |
| 21-1c | Témoin | Eau NaCI 0.9% | 80.2 |
| 21-2c | Plasma | Eau NaCI 0.9% | 51.4 |
| 21-3 | Plasma+PVP | Eau NaCI 0.9% | 56.1 |
| 21-4c | Plasma+HPMC | Eau NaCI 0.9% | 58.7 |

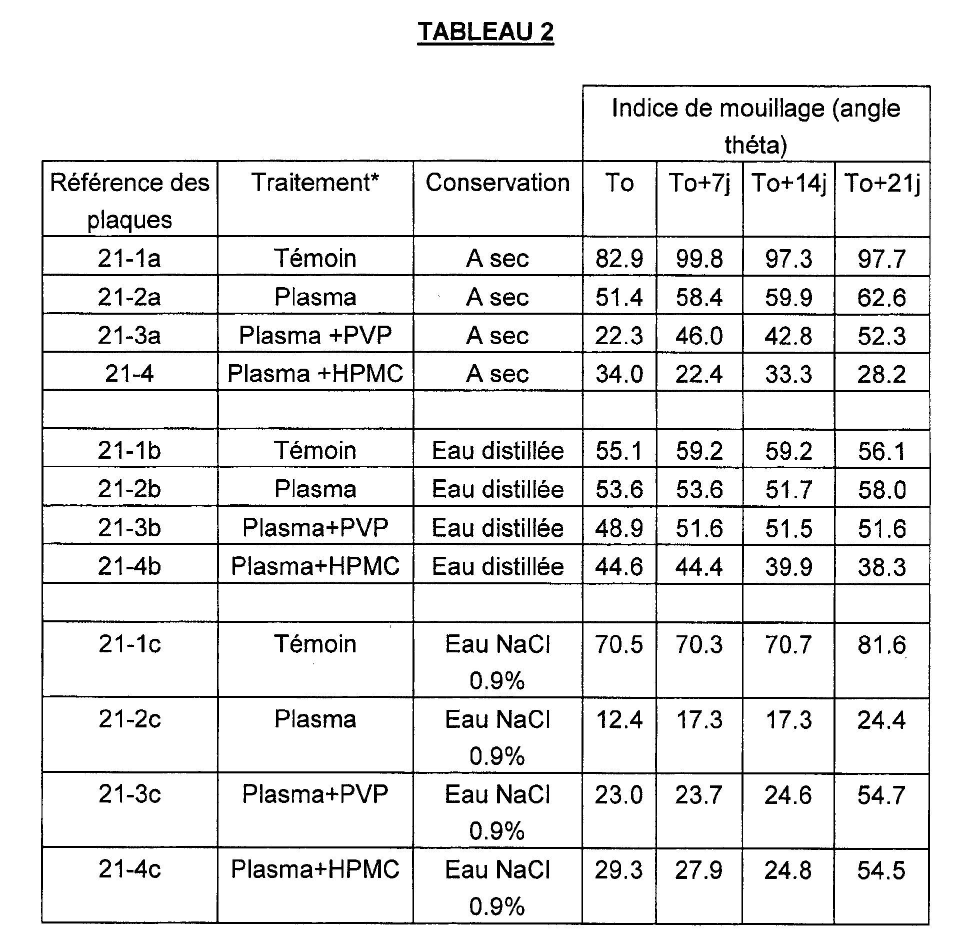

## Revendications

1. Prothèse dentaire comprenant un premier matériau polymère de base, contenant des atomes d'hydrogène, à la surface duquel les atomes d'hydrogène sont remplacés partiellement par des groupements -OH, -COOH, -C=O, -OOH, -NH₂, C=NH et/ou -CONH₂, **caractérisée en ce que** ladite surface du premier matériau polymère est recouverte au moins sur certaines zones d'une couche d'un second matériau polymère à caractère hydrophile, lié de manière non covalente au premier matériau polymère.

2. Prothèse dentaire selon la revendication 1, **caractérisée en ce que** le second matériau polymère recouvre exclusivement l'intrados prothétique de ladite prothèse.

3. Prothèse dentaire selon l'une des revendications 1 ou 2, **caractérisée en ce que** le second matériau polymère recouvre à la fois l'intrados et l'extrados prothétique.

4. Prothèse dentaire selon l'une quelconque des revendications 1 à 3, **caractérisée en ce que** la couche du second matériau polymère possède une épaisseur comprise entre environ 2 et 500 µm, préférentiellement entre environ 5 et 100 µm et de manière tout à fait préférée entre environ 5 et 50 µm.

5. Prothèse dentaire selon l'une des revendications 1 à 4, caractérisée en ce le second matériau polymère à caractère hydrophile est au moins partiellement soluble dans l'eau ou dans un solvant miscible à l'eau.

6. Prothèse dentaire selon l'une des revendications 1 à 5, **caractérisée en ce que** le second matériau polymère se gonfle et devient déformable au contact de l'eau.

7. Prothèse dentaire selon l'une des revendications 1 à 6, **caractérisée en ce que** le second matériau polymère est choisi parmi les celluloses et leurs dérivés, les polyacrylamides et leurs copolymères, la polyvinylpyrrolidone (PVP) et ses copolymères, les copolymères d'acétate de vinyle et d'alcool vinylique, les polyéthylène-glycols, les polypropylène-glycols, les polyacrylates hydrophiles, les polyméthacrylates hydrophiles, les polyosides et les chitosans.

8. Prothèse dentaire selon l'une des revendications 1 à 7, **caractérisée en ce que** le remplacement des atomes d'hydrogène à la surface du premier matériau polymère est localisé sur une épaisseur de 10 à 30 nm.

9. Prothèse dentaire selon l'une des revendication 1 à 8, **caractérisée en ce qu'**il s'agit de préférence d'une prothèse définitive, la prothèse pouvant également être à pose immédiate.

10. Procédé d'obtention de prothèses dentaires, comprenant une étape de traitement par un plasma d'oxygène, d'argon, de gaz carbonique ou d'azote d'un premier matériau polymère, préalablement obtenu sous la forme appropriée à son utilisation, **caractérisé en ce que** la surface du premier matériau polymère traitée par plasma est recouverte d'un second matériau polymère à caractère hydrophile, qui se lie de manière non covalente au premier matériau polymère.

11. Procédé d'obtention de prothèses dentaires selon la revendication 10, **caractérisé en ce que** le second matériau polymère est apporté à la surface du premier matériau polymère par trempage ou encore par application au pinceau ou au pistolet.

12. Procédé d'obtention de prothèses dentaires selon la revendication 11, **caractérisé en ce que** le second matériau polymère est apporté à la surface du premier matériau polymère sous la forme d'une solution ou suspension aqueuse dudit second matériau polymère à une concentration comprise entre environ 0,5% et environ 5%, préférentiellement entre environ 1% et environ 3% en poids total de la solution ou suspension aqueuse.
